# EUROPÄISCHE PATENTSCHRIFT

(11) **EP 1 722 695 B1**
(45) Veröffentlichungstag und Bekanntmachung des Hinweises auf die Patenterteilung: **15.05.2013**
(21) Anmeldenummer: 05715973.3
(22) Anmeldetag: 11.03.2005
(51) Int. Cl.: A61B 17/22, A61F 2/01

(54) **VORRICHTUNG ZUR REKANALISIERUNG EINES HOHLRAUMS, ORGANWEGS ODER GEF SSES**
DEVICE FOR RECHANNELLING A CAVITY, ORGAN PATH OR VESSEL
DISPOSITIF DE RECANALISATION D'UNE CAVITE, D'UN CANAL ORGANIQUE OU D'UN VAISSEAU

(30) Priorität: 11.03.2004 DE 102004012351
(43) Veröffentlichungstag der Anmeldung: 22.11.2006
(73) Patentinhaber: pfm medical ag, 50966 Köln (DE)
(72) Erfinder: FREUDENTHAL, Franz, La Paz (BO); SCHMITZ-RODE, Thomas, 52070 Aachen (DE)
(74) Vertreter: Ring & Weisbrodt
(86) Internationale Anmeldenummer: PCT/EP2005/002608
(87) Internationale Veröffentlichungsnummer: WO 2005/087117

(56) Entgegenhaltungen:
- EP-A- 1 306 063
- WO-A-01/05311
- WO-A-02/071975
- WO-A-03/009880
- DE-B- 1 069 823
- US-A- 5 800 514
- US-A- 5 902 317

## Beschreibung

Die Erfindung betrifft eine Vorrichtung zur Rekanalisierung eines von zumindest einem komprimierbaren Verschlussobjekt, insbesondere einem Thrombus oder Embolus, zumindest teilweise verschlossenen Hohlraumes, Organwegs oder Gefäßes im menschlichen oder tierischen Körper mit zumindest einem komprimierbaren und selbstexpandierenden Stent mit einem distalen und proximalen Ende.

Auch im Zeitalter der prophylaktischen Antikoagulation stellt die akute Lungenembolie ein häufiges und oft lebensbedrohliches Ereignis dar. Die Standardtherapie besteht bei massiver Lungenembolie in der medikamentöse Thrombolyse mit Streptokinase, Urokinase oder Gewebsplasminogenaktivator. Ziel ist dabei die Rekanalisation des betroffenen Gefäßes. Nach Anwendung dieser Standardtherapien zeigen Kontrollen mit bildgebenden Verfahren, beispielsweise durch Echokardiographie, Angiographie, Computertomographie, lediglich eine geringe Rekanalisation nach Thrombolyse. Trotz Hochdosisthrombolyse verstirbt daher ein Teil der Patienten an Rechtsherzversagen.

Alternativ kann bei kontraindizierter Thrombolyse, beispielsweise intrakranieller Verletzung oder nach Operationen, oder bei fehlgeschlagener Thrombolyse das Embolusmaterial operativ ausgeräumt werden. Der Eingriff unter Einsatz der Herz-Lungen-Maschine ist sehr belastend für den Patienten und mit einer hohen Letalität behaftet.

Es wurden daher verschiedene Vorrichtungen entwickelt, um das Ausräumen und Rekanalisieren der Gefäße zu erleichtern. Von Greenfield wurde 1964 ein mechanisches Instrument mit endoskopartiger Steuerung entwickelt, das pulmonale Emboli über eine venöse Punktionsstelle nicht-operativ extrahiert. Aufgrund seiner umständlichen Handhabung hat sich dieser Saugkatheter nicht durchgesetzt. Von Günther und Schmitz-Rode wurde 1991 ein Hochgeschwindigkeitskathetersystem zum Fragmentieren pulmonaler Emboli entwickelt, das sich jedoch aufgrund seiner technischen Komplexität und nicht ausreichenden pulmonalen Steuerbarkeit des Katheters ebenfalls nicht durchsetzten konnte. Ein von Günther und Schmitz-Rode 1995 entwickelter modifizierter Pigtail-Katheter wird rotierbar im embolischen Verschluss bewegt und bewirkt dadurch eine grobe Fragmentation des Embolusmaterials. Hierbei kann jedoch lediglich das weiche und frische Embolusmaterial fragmentiert werden. Bei festeren organisierten Emboli versagt der modifizierte Pigtail-Katheter. Für den Fall von zentral bis peripher mit Embolusmassen angefüllten Pulmonalarterien besteht wenig Aussicht auf einen Rekanalisierungserfolg durch Fragmentation, da die Fragmente nicht nach peripher abschwimmen können.

Diese Nachteile treten bei der US 2002/0095161 A1 nicht auf. Bei dieser Vorrichtung zur Extraktion von beispielsweise ureteralen Steinen werden diese in einem Korb eingefangen, der eine große Öffnung aufweist, die über die Hälfte der Oberfläche des Korbes einnimmt und durch die Steine und Steinfragmente in das Innere des Korbes eintreten können. Außerdem weist der Korb relativ schmale Öffnungen auf, die zum Zurückhalten der Steine und Steinfragmente geeignet sind. Als nachteilig erweist sich hierbei, dass der Einfangvorgang trotz der Möglichkeit, den Korb zu rotieren, relativ mühsam ist und der einzufangende Stein nicht problemlos in den Korb gelangt.

Ein ähnliches Problem tritt bei der Extraktionsvorrichtung nach der US 5,779,716 auf, bei der ein sackartiger Fangkorb mit einem Bügeldraht an seinem proximalen Ende versehen ist, wobei der Bügeldraht die proximale Öffnung des sackartigen Fangkorbs offen hält, um den Einfangvorgang zu unterstützen.

Im Stand der Technik sind außerdem Extraktionsvorrichtungen mit gewendelten Drähten bekannt, zwischen denen ein Stein oder ein anderer Fremdkörper eingefangen werden kann. Ein Beispiel dafür gibt die WO 99/47054. Hierbei steht zu befürchten, dass der Stein während des Zurückziehens beim Bergungsvorgang wieder aus den Schlingen herausrutscht. Dies gilt auch für die in der WO 01/05311 A1 offenbarte Extraktionsvorrichtung und die in der US 2002/0026203 A1 offenbarte Extraktionsvorrichtung.

Zum Entfernen von Thromben aus dem Vaskularsystem ist es aus der US 5,419,774 A bekannt, in der Extraktionsvorrichtung an deren distalem Ende eine Kammer vorzusehen, in die der Thrombus durch Unterdruck gesogen wird. In der Kammer befindet sich eine Trennvorrichtung, die den Teil des Thrombus abtrennt, der sich in der Kammer befindet. Ein unter Druck stehendes Fluid wird zugeführt, um den Thrombus und das sich bei diesem sammelnde Blut abzutransportieren. Der Aufbau der Vorrichtung ist jedoch relativ aufwendig, da zum einen eine Einrichtung zum Aufbringen eines Unterdrucks vorgesehen sein muss und zum anderen eine Einrichtung zum Zuführen einer unter Druck stehenden Flüssigkeit. Außerdem muss eine Einrichtung zum Trennen des Thrombus vorgesehen sein. Diese Nachteile betreffen auch den Katheter zum Bearbeiten und Entfernen von weichen und harten Substanzen zur Verwendung in der invasiven Mikrochirurgie und Gefäßbehandlung gemäß der DE 197 34 890 C1. Der Anwendungsbereich ist hierbei das Entfernen von beispielsweise Gewebe oder Gallensteinen.

Zum Entfernen von Thromben ist im Stand der Technik außerdem aus der US 2002/0026211 A1 bekannt, eine Vorrichtung und ein Verfahren zum Filtern von Emboli oder Entfernen von Thromben aus einem Gefäß vorzusehen, bei dem die Vorrichtung einen Vaskularfilter zum Ergreifen der Emboli und optional ein Thrombektomie-Element zum Entfernen des Thrombus enthält. Der Vaskularfilter enthält einen Tragring mit einem oder mehreren Gelenkbereichen, die nahe einem distalen Ende eines Führungsdrahtes befestigt sind, sowie ein blutdurchlässiges Säckchen, das an dem Tragring befestigt ist. Der Tragring bildet die Öffnung des blutdurchlässigen Säckchens und hält diese offen. Es können zwei hintereinander angeordnete Säckchen vorgesehen sein, deren Öffnungen in dieselbe Richtung, nämlich nach proximal zeigen. Mit dem ersten Vaskularfilter wird der Thrombus eingefangen und mit dem zweiten die verbleibenden Emboli. Beide Vaskularfilter werden in ein Rohr bzw. einen Katheter zurückgezogen, zusammen mit dem Thrombus und den Emboli. Diese Vorrichtung erweist sich als nachteilig aufgrund der Tragringe in Verbindung mit dem blutdurchlässigen Säckchen, da deren Herstellungsaufwand aufgrund der in den Tragringen vorgesehenen Gelenke recht hoch ist. Außerdem ist die Verletzungsgefahr des Gefäßes, aus dem der Thrombus bzw. die Emboli entfernt werden sollen, aufgrund der Verwendung eines Tragrings recht hoch, da dieser im Vergleich zu dem Gefäß starr und unbeweglich ist und gegebenenfalls an der Gefäßinnenwand schabt.

Eine weitere Vorrichtung zum Entfernen von Thromben ist aus der US 5,011,488 A bekannt. Hierbei enthält ein Vaskularkathetersystem ein äußeres flexibles Rohr, ein inneres flexibles Rohr, das in dem Lumen des äußeren flexiblen Rohres angeordnet ist, und einen expandierbaren Körper, der an einem distalen Ende eines dritten flexiblen Rohres angeordnet ist, das seinerseits in dem Lumen des inneren flexiblen Rohres angeordnet ist. Das innere Rohr enthält eine expandierbare Spitze, die sich öffnen kann, um im Wesentlichen den Querschnitt eines Blutgefäßes einzunehmen. Der expandierbare Körper wird durch das Thrombusgebiet hindurch ausgestreckt und expandiert. Das zu entfernende Thrombusmaterial befindet sich dann zwischen den beiden expandierten Teilen, wobei die expandierte Spitze ein aufblasbarer Körper ist, der sich an der Innenwand des Gefäßes anlegt und beim Zurückziehen in das Rohr das Thrombusmaterial von der Gefäßinnenwand zu dem expandierten Körper hin abschabt und mitnimmt. Der aufblasbare Körper oder Ballon passt dabei in den expandierten offenen Körper hinein und ist entsprechend diesem kegelstumpfförmig. Der expandierte Körper weist eine Mehrzahl von Federelementen auf, die nach dem Herausschieben aus dem Rohr zu einem Öffnen der expandierbaren Spitze führen. Aufgrund der Kegelstumpfform wird der expandierte Körper beim Zurückziehen in das Rohr wieder zusammengeschoben. Dasselbe geschieht mit dem dann innen liegenden aufblasbaren Körper oder Ballon. Diese Vorrichtung erweist sich als nachteilig, da bei dem Zurückziehen der beiden Körper das sich zwischen diesen befindende Thrombusmaterial insbesondere beim Komprimieren des aufblasbaren Körpers wieder austreten kann, also nicht sicher zwischen dem aufblasbaren Körper und dem expandierbaren offenen Kegelstumpfkörper festgehalten wird.

Aus der WO 00/51505 A1 ist eine Extraktionsvorrichtung mit nur einem ballonartig aufweitbaren distalen Abschnitt bekannt, das verschlungene Drähte aufweist, die mit einem Gewebe belegt sind. Zum Ausräumen eines Gefäßes schabt das aufgeweitete Ende an der Gefäßwand entlang, und das Gewebe verhindert ein Eindringen von abgeschabten Fremdkörpern in die Vorrichtung bzw. den aufgeweiteten Abschnitt.

Die DE 692 28 326 T2 offenbart eine Extraktionsvorrichtung, bei der ein flexibles Schleifenteil mit einem Netz bespannt und an seinem distalen und proximalen Ende auf einem Zugdraht befestigt ist. Das Netz kann durch Verschieben des Schleifenteils entlang dem Zugdraht eine offene und eine geschlossene Form annehmen. Hierdurch kann ein Fremdkörper in dem verschlungenen mit Netz bespannten Schlingenteil eingefangen werden.

Aus der WO 00/53120 A1 ist eine Extraktionsvorrichtung bekannt, bei der zwei Fangkörbe in Ausrichtung der Öffnungen der Fangkörbe zueinander vorgesehen sind. Der distale Fangkorb ist an einem Stab befestigt und innerhalb des anderen Fangkorbs in diesen hineinziehbar angeordnet. Der distale Fangkorb weist ein distales zusammengezogenes Ende auf, wohingegen der proximale Fangkorb ein proximales zusammengezogenes Ende aufweist.

Aus der WO02/064065 und US2002/0035394 ist eine Vorrichtung zum Rekanalisieren von Gefäßen unter Verwendung eines Stents mit zylindrischer Form bekannt. Der zylindrische Stent wird komprimiert und über einen Katheter durch einen Embolus hindurch geschoben und in diesem expandiert. Hierdurch drückt er allseitig mit seiner Mantelfläche das Embolusmaterial gegen die Gefäßwand und sorgt dadurch für eine Rekanalisation des Gefäßes. Gerade bei gekrümmten Gefäßverläufen kann es bei dem zylindrischen Stent zu einem ungewollten Vorbeirutschen von Embolusmaterial kommen, das peripher zu weiteren Verschlüssen führt.

Der vorliegenden Erfindung liegt nun die Aufgabe zugrunde, die vorstehend genannten Nachteile des Standes der Technik zu vermeiden und eine Vorrichtung zur Rekanalisierung von Hohlräumen und/oder Organwegen im menschlichen oder tierischen Körper vorzusehen, bei der keine Bergung von komprimierbaren einen Hohlraum, Organweg oder ein Gefäß verschließenden Objekten, insbesondere Thromben, mehr erforderlich ist, wobei dennoch eine effektive nicht-operative Rekanalisierung gerade bei teilorganisierten Emboli und bei ausgedehnter Embolisierung der Lungenstrombahn ermöglicht werden soll.

Die Aufgabe wird durch eine Extraktionsvorrichtung nach dem Oberbegriff des Anspruchs 1 dadurch gelöst, dass der zumindest eine Stent im Wesentlichen glockenförmig mit einem aufgeweiteten distalen Ende und einem eingeschnürten proximalen Ende ausgebildet ist. Weiterbildungen der Erfindung sind in den abhängigen Ansprüchen definiert.

Dadurch wird eine Vorrichtung zur Rekanalisierung eines Hohlraums, Organwegs oder Gefäßes im menschlichen oder tierischen Körper geschaffen, mit deren Hilfe die vorhandenen komprimierbaren Verschlussobjekte an die jeweilige Wand des Hohlraumes, Organwegs, Gefäßes gepresst werden können, um sie dort auszudrücken. Der Stent entfaltet sich beim Absetzen im Bereich des zu komprimierenden Objekts selbständig. Vorzugsweise besteht er daher aus einem Formgedächtnismaterial. Aufgrund der Glockenform des Stents kann ein Eindringen von Teilen des komprimierbaren Verschlussobjekts, insbesondere von Embolusmaterial, in den Stent hinein sicher vermieden werden. Im Unterschied zu der WO02/064065 A2 bzw. der US 2002/0035394 A1 kann dabei der Stent sogar kürzer sein als das zu komprimierende Objekt, ohne dass die Gefahr besteht, dass größere Abschnitte des komprimierbaren Objekts in den Stent hinein und durch diesen hindurch an eine andere Stelle im menschlichen oder tierischen Körper gelangen, wo diese Abschnitte wiederum einen ungewollten Verschluss eines Hohlraums/Organwegs bzw. Gefäßes verursachen können. Der in der WO02/064065 A2 beschriebene Stent ist nach dem Entfalten zylindrisch, so dass dieser gegebenenfalls auch bei starker Blutströmung von dem Ort, an dem er abgesetzt wurde, weggespült werden kann. Aufgrund der Glockenform gemäß der vorliegenden Erfindung ist ein besserer Halt in dem Hohlraum/Organweg/Gefäß im menschlichen oder tierischen Körper möglich. Hierbei ist das distale Ende des zumindest einen Stents aufgeweitet und das proximale Ende des zumindest einen Stents eingeschnürt. Gerade im distalen Endbereich des Stents ist aufgrund des leicht auskragenden Endes eine besonders gute Abstützung an einer Wand von Hohlraum/Gefäß/Organweg möglich. Auch im proximalen Bereich des Stents kragt dieser aufgrund seiner Glockenform aus, so dass auch hier ein guter Halt gerade in Organwegen bzw. Gefäßen möglich ist. Zwischen den beiden auskragenden Endbereichen des erfindungsgemäßen Stents kann das zu komprimierende Verschlussobjekt außerdem sehr gut festgehalten werden, so dass dieses nicht ungewollt beim Platzieren des Stents und danach weggleiten kann. Auch das Folgen einer Krümmung eines Organwegs, Gefäßes oder Hohlraums, wie beispielsweise eines Aortenbogens, ist durch die Glockenform leichter und effektiver möglich, wobei vor allem das auskragende distale Ende und ein eingeschnürter proximaler Bereich sich für das Verhindern eines Durchrutschens eines zu komprimierenden Verschlussobjekts als vorteilhaft erweisen. Eine optimale Anpassung an die Anatomie des jeweiligen Organwegs, Gefäßes oder Hohlraums, insbesondere des rechten und linken Pulmonalarterien-Hauptastes, der sich stark verengt, also konisch ausgebildet ist, ist durch die Glockenform möglich. Außerdem kann Blut proximal besser durch die aufgrund der Glockenform aufgeweiteten Maschen einströmen, wodurch kleinere Partikel schnell und effektiv mitgerissen und weggeschwemmt werden können.

Im Stand der Technik ist eine solche Glockenform eines Stents nicht für die Zwecke einer Rekanalisierung eines Hohlraumes oder Organwegs oder Gefäßes bekannt. Vielmehr offenbart beispielsweise die EP 0 836 450 B1 zwar einen sich selbst entfaltenden Stent bzw. Carvafilter mit einer Hantelform, einer halben Haltelform oder einer Kugelsegmentform. Diese stellen jedoch keine Glockenform dar, sondern vielmehr stets sich zumindest an einem Ende nach Art eines Kegels aufweitende Gebilde. Gemäß der WO03/075799 wird auch lediglich ein Endovaskular-Stent-Implantat in Form eines Glockenbodens offenbart, wobei bei dieser Vorrichtung beinartige Verlängerungen vorgesehen sind, so dass eine Glockenform ebenfalls nicht erkannt werden kann. Diese Vorrichtungen dienen lediglich zum Stützen eines Gefäßes. Aus der WO01/72239 A2 ist wiederum ein hantelförmiges Implantat bekannt, das aus einem geschnittenen Röhrchen gebildet ist. Eine Verwendung zur Rekanalisierung von Organwegen oder Gefäßen ist in dieser Druckschrift nicht offenbart und das hantelförmige Implantat auch für eine solche Anwendung nicht geeignet, insbesondere aufgrund seiner mittig eingeschnürten Form. Die US2002/0007222 A1 offenbart Stützeinrichtungen zur Implantation in Gefäßen, wobei diese Stützeinrichtungen bzw. Stents einen mittleren ausgebauchten Bereich und endseitige auskragende Bereiche aufweisen, jedoch keine Glockenform mit lediglich einem aufgeweiteten distalen Ende und einem eingeschnürten proximalen Ende und einem dazwischen angeordneten gegenüber dem aufgeweiteten distalen Ende verengten Bereich.

Durch das Einschnüren des proximalen Endes kann dieses besonders gut in einem Katheter gehalten werden. Das eingeschnürte proximale Ende des Stents kann vorteilhaft außerdem mit einem Hülsenelement versehen sein, um ein ungewolltes Aufweiten dieses proximalen Endes sicher zu vermeiden und ein gutes Befestigen am Katheter zu ermöglichen. Ein Öffnen des proximalen Endes und die Gefahr eines Eindringens eines Abschnitts des zu komprimierenden Objekts in den Stent kann ebenfalls sicher vermieden werden.

Der erfindungsgemäße Stent ist vorzugsweise einseitig an dem (Träger)katheter befestigt. Der übrige Körper des Stents kann entfaltet, vorzugsweise jedoch nicht vollständig vom Katheter gelöst werden. Hierdurch ist ein problemloses und schnelles Entfernen des Stents vom Implantationsort möglich. Im Unterschied hierzu werden bei den Stents bzw. Vorrichtungen des Standes der Technik, wie sie vorstehend beschrieben sind, diese stets vollständig vom Katheter gelöst und können daher nicht oder zumindest nicht ohne Weiteres wieder von dem Implantationsort im Patienten entfernt werden.

Anstelle des Vorsehens eines Hülsenelementes und des Befestigens des Stents am Katheter können zum Befestigen des Stents ein oder mehrere drahtartige Befestigungselemente vorgesehen werden, insbesondere Einzeldrähte oder eine Drahtlitze oder gegebenenfalls mehrere Drahtlitzen. Diese sind ebenfalls mit dem Stent so fest verbunden, dass ein schnelles und einfaches Entfernen des Stents vom Implantationsort möglich ist. Vorzugsweise ist das proximale Ende des Stents mit zumindest teilweise verdrillten Schlaufen ausgebildet, die insbesondere aus der Netzstruktur des Stents herausgeführt sind. Durch das oder die drahtartigen Befestigungselemente können die Schlaufen erfasst und das drahtartige Befestigungselement/die drahtartigen Befestigungselemente in einen Katheter hineingeführt werden. Über die drahtartigen Befestigungselemente ist ein Halten des Stents am Implantationsort leichter möglich als über einen mit dem Stent fest verbundenen Katheter, da die drahtartigen Befestigungselemente sich leichter biegen und damit die Form des Organwegs annehmen können. Zum späteren Entfernen des Stents muss lediglich an den drahtartigen Befestigungselementen in Richtung proximal gezogen werden. Der an seinem proximalen Ende über die Schlaufen und drahtartigen Befestigungselemente eingeschnürte Stent folgt dann der Bewegung der drahtartigen Befestigungselemente zum Deplatzieren oder Entfernen des Stents.

In einer Ausführungsform können alle, in einer anderen auch lediglich ein Teil der Schlaufen von einem drahtartigen Befestigungselement zusammengefasst werden. Anstelle eines separaten drahtartigen Befestigungselementes oder mehrerer separater drahtartiger Befestigungselemente kann zumindest eine zumindest teilweise verdrillte Schlaufe aus der Netzstruktur des Stents länger als die übrigen Schlaufen ausgebildet und durch diese hindurchgefädelt sein zum Ausbilden eines drahtartigen Befestigungselements für den Stent. Hierbei erweist es sich als besonders vorteilhaft, die als drahtartiges Befestigungselement ausgebildete Schlaufe im Bereich zwischen ihrem verdrillten Abschnitt und dem durch die benachbarten Schlaufen gefädelten Abschnitt mit einem Hülsenelement zum Stabilisieren der Verbindung zu versehen. Durch das Hülsenelement werden dabei vorzugsweise beide Enden der Schlaufe gezogen, um eine ausreichend stabile Verbindung zu schaffen. Die als drahtartiges Befestigungselement ausgebildete Schlaufe kann endseitig geschlossen oder offen ausgebildet sein, wobei dieses proximale Ende der Schlaufe ohnehin nach exkorporal geführt wird, um den Stent am Implantationsort bewegen bzw. dort deplatzieren zu können. Der proximale Teil der als drahtartiges Befestigungselement ausgebildeten Schlaufe kann entweder direkt in einen Katheter nach dem Durchfädeln der benachbarten Schlaufen geführt oder vor dem Einfügen in den Katheter erneut zu Schlingen oder Schlaufen gelegt werden, um einen noch besseren Halt an dem proximalen Ende des Stents zu gewähren.

Bevorzugt ist ein zwischen dem distalen und proximalen Ende des zumindest einen Stents angeordneter Bereich gegenüber der Öffnungsweite des distalen Endes verengt und ein zwischen dem verengten Bereich und dem eingeschnürten proximalen Ende angeordneter Abschnitt gegenüber dem verengten Bereich aufgeweitet. Aufgrund des Vorsehens des verengten Bereichs zwischen dem aufgeweiteten proximalen Abschnitt und dem aufgeweiteten distalen Ende kann das zu komprimierende Objekt einerseits besonders gut gehalten werden und wird andererseits in diesen mittleren verengten Bereich hineingedrängt, so dass das Komprimieren hierdurch ebenfalls unterstützt wird.

Vorzugsweise weist der Stent eine Netzstruktur und/oder eine Vielzahl von Öffnungen mit einer Öffnungweite, die geringer als die Abmessungen des zu komprimierenden Objekts ist, auf. Besonders bevorzugt besteht der Stent aus einem Geflecht und/oder Gewebe und/oder Gelege, insbesondere einem Drahtgeflecht und/oder Drahtgewebe und/oder Drahtgelege. Alternativ kann der zumindest eine Stent aus einem zumindest über einen Teil seiner Länge geschlitzten Rohr bestehen. Durch Vorsehen einer großen Anzahl von Öffnungen bzw. einer Netzstruktur kann Flüssigkeit aus dem zu komprimierenden Verschlussobjekt herausgedrückt und auf der Innenseite des Stents abgeführt werden. Feste Bestandteile des Objekts verbleiben hingegen auf der Außenseite des Stents zwischen diesem und der Wandung des Hohlraums bzw. Organwegs bzw. Gefäßes. Diese festen Bestandteile können entweder nach dem Entfernen des Stents aus dem Hohlraum bzw. Organweg bzw. Gefäß über eine entsprechend durch diesen hindurchgehende Körperflüssigkeit, insbesondere Blut, abgeführt werden. Alternativ oder zusätzlich kann eine Absaugung der festen Bestandteile des zu komprimierenden Verschlussobjekts vorgesehen werden. Zu diesem Zweck ist vorzugsweise eine Absaugeinrichtung zum Absaugen von festen Bestandteilen des zu komprimierenden Objekts oder Objektteilen vorgesehen, insbesondere eine Kanüle oder dergleichen rohrförmige Einrichtung, die in die Nähe des zumindest einen in dem Hohlraum, Organweg oder Gefäß abgesetzten Stents führbar und mit einem Unterdruck beaufschlagbar ist. Über eine solche Absaugeinrichtung können dann die festen Bestandteile des Objekts bzw. gegebenenfalls kleinere abgetrennte Objektteile aus dem menschlichen oder tierischen Körper entfernt werden.

Bevorzugt ist das Material des zumindest einen Stents mit einer Beschichtung versehen, insbesondere einer biokompatiblen Oberflächenbeschichtung, Heparin, einer Carbonisierung von Nitinol, einer nanotechnologischen Beschichtung, röntgendichten Partikeln, einer einen Wirkstoff freisetzenden Beschichtung, einer insbesondere mikroporösen biotechnologischen oder einer anderen Beschichtung. Durch Vorsehen einer solchen Beschichtung kann das Auflösen des zu komprimierenden Objekts noch weiter unterstützt werden. Bei Vorsehen einer die Oberfläche des Stents aufrauenden Beschichtung ist außerdem ein besseres Festhalten des zu komprimierenden Objekts durch den Stent möglich. Hierbei wird vorzugsweise jedoch darauf geachtet, dass eine Beschädigung der jeweiligen Wandung des Hohlraumes bzw. Organwegs bzw. Gefäßes im menschlichen oder tierischen Körper, in dem der Stent angeordnet wird, vermieden wird.

Vorzugsweise besteht der zumindest eine Stent aus einem nicht thrombogenen biokompatiblen Formgedächtnismaterial, insbesondere einem Metall oder einer Metalllegierung, insbesondere einem Edelstahl oder Nitinol oder einem anderen biokompatiblen Material, wie insbesondere einem Kunststoff. Da der Stent zumindest einige Stunden bis Tage im menschlichen oder tierischen Körper verbleibt, erweist sich die Verwendung eines biokompatiblen Materials zum Vermeiden von Abstoßungseffekten und allergischen Reaktionen als besonders vorteilhaft. Um dem Stent die Glockenform einzuprägen, ihn nachfolgend komprimieren und sich selbstexpandierend öffnen lassen zu können, wird vorteilhaft ein Formgedächtnismaterial gewählt.

Für den Rekanalisationsvorgang erweist es sich als besonders vorteilhaft, den zumindest einen Stent im Unterschied zur Lehre der WO02/064065 A2 auf einer Seite des zu komprimierenden Objekts vorbeizuführen und dieses nur einseitig gegen die jeweilige Wandung des Hohlraums/Organwegs/Gefäßes zu pressen. Gerade bei Lungenembolien mit verschließendem Embolus kann hierdurch eine besonders effektive Rekanalisation erfolgen, wobei nur eine exzentrische Rekanalisierung des Gefäßes/Hohlraums/Organwegs erfolgt, was das Gefäß etc. weniger belastet als eine allseitige Aufweitung. Der ursprüngliche Durchflussdurchmesser kann direkt wieder zur Verfügung gestellt werden. Vorzugsweise ist der Durchmesser des expandierenden Stents daher im Wesentlichen gleich dem oder etwas größer als der Durchmesser des zu rekanalisierenden Hohlraums/Organwegs/Gefäßes dimensioniert. Hierdurch kann der Stent das zu komprimierende Objekt stetig quetschen und in seine flüssigen und festen Bestandteile trennen, auch bei sich verkleinerndem Objekt.

Eine Anwendungsvariante der Vorrichtung zur Rekanalisierung gemäß der vorliegenden Erfindung ist die eines Aortenfilters. Gerade in der heutigen Zeit müssen immer mehr Patienten aufgrund eines Thrombus behandelt werden. Durch bildgebende Verfahren kann festgestellt werden, dass häufiger Thrombosen im linken Vorhof und der linken Herzkammer zu finden sind. Dies betrifft immer öfter vor allem ältere Patienten. Auch nimmt die Zahl der Patienten mit Herzrhythmusstörungen zu. Bei Eingriffen und Operationen steigt das Thromboserisiko, insbesondere auch bei Ablationen, Implantationen und der Verwendung einer Herzlungenmaschine. Auch ansonsten werden immer mehr Patienten mit einem erhöhten Thromboserisiko diagnostiziert, z.B. im Zusammenhang mit komplizierten Schwangerschaften, Atrium Systemdefekt (ASD), PFO, Aneurismen bei Chagas-Krankheit, Tumoren etc. Ein Aortenfilter kann hierbei zum Schutz beider Arteriae Carotis vor einer Thrombose, also vor einem Schlaganfall und zum Schutz beider Koronarien vor einer Thrombose, also vor einem Herzinfarkt, verwendet werden. Thromben können gefangen und entfernt werden, z.B. durch Einklemmen zwischen Gefäßwand und Stent und nachfolgendes Absaugen. Bei Verwendung des Aortenfilters wird vorteilhaft darauf geachtet, dass das beim Filtern entstehende Material sowie der Aortenfilter selbst nicht thrombogen sind und insbesondere keine hochgefährlichen Mikroemboli gebildet werden, die wiederum das arterielle System schädigen könnten.

Die Vorrichtung kann im Wesentlichen so lang ausgebildet sein wie ein Aortenbogen, in dem sie angeordnet wird oder werden soll, zum Schutz von Arteria Carotis und Koronarien. Alternativ kann die Vorrichtung kürzer als ein Aortenbogen, in dem sie angeordnet wird oder werden soll, ausgebildet und mit einem wenig oder gar nicht aufgeweiteten Ende versehen sein zum Schutz beider Arteriae Carotis. Wird die Vorrichtung im Wesentlichen so lang ausgebildet wie ein Aortenbogen, hält sich das auskragende bzw. aufgeweitete distale Ende am Ende des Aortenbogens in dem dort ebenfalls ausgestülpten Abschnitt vorzugsweise fest. Ist hingegen die Vorrichtung kürzer als der Aortenbogen ausgebildet, endet also im Implantationszustand mitten innerhalb des Aortenbogens, kann zwar grundsätzlich das distale Ende ebenfalls aufgeweitet ausgebildet sein, es reicht jedoch ebenso aus, das distale Ende lediglich geringfügig aufzuweiten oder sogar gar nicht aufzuweiten, wobei dennoch üblicherweise ein guter Halt aufgrund der Krümmung entsprechend dem Aortenbogen innerhalb von diesem gewährleistet werden kann, wenn die Vorrichtung als Aortenfilter eingesetzt wird.

Zur näheren Erläuterung der Erfindung werden im Folgenden Ausführungsbeispiele anhand der Zeichnungen näher beschrieben. Diese zeigen in:
- Figur 1: eine Seitenansicht einer ersten Ausführungsform einer erfindungsgemäßen Vorrichtung mit einem glockenförmigen Stent,
- Figur 2a-2f: Prinzipskizzen einer Lungenembolie mit verschließendem Embolus und der Schritte zur Rekanalisation des Organwegs,
- Figur 3: eine Seitenansicht einer zweiten Ausführungsform einer erfindungsgemäßen Vorrichtung mit einem glockenförmigen Stent,
- Figur 4a-c: Detailansichten einer ersten Ausführungsform einer proximalen Befestigung des Stents durch drahtartige Befestigungselemente,
- Figur 5a-c: eine zweite Ausführungsform einer proximalen Befestigung eines erfindungsgemäßen Stents über zwei drahtartige Befestigungselemente,
- Figur 6a-c: Detailansichten einer weiteren Ausführungsform zur proximalen Befestigung eines erfindungsgemäßen Stents durch ein mit einer proximalen Schlaufe einteiliges Befestigungselement mit aufgefügtem Hülsenelement,
- Figur 7a-c: eine weitere Ausführungsform einer proximalen Befestigung eines erfindungsgemäßen Stents über eine als drahtartiges Befestigungselement ausgebildete aus der Netzstruktur des Stents herausgezogene Schlaufe mit aufgefügtem Hülsenelement,
- Figur 8: eine Prinzipskizze eines langen glockenförmigen Stents nach Implantation in einem Aortenbogen,
- Figur 9: eine Prinzipskizze eines kürzeren erfindungsgemäßen Stents nach Applikation in einem Aortenbogen,
- Figur 10: eine Prinzipskizze des kurzen erfindungsgemäßen Stents gemäß Figur 9, und
- Figur 11: eine Prinzipskizze des langen glockenförmigen erfindungsgemäßen Stents gemäß Figur 8.

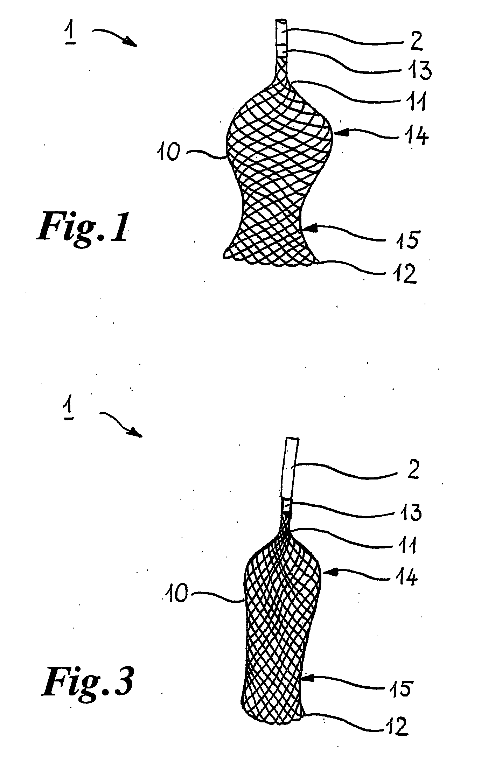

Figur 1 zeigt eine Seitenansicht einer ersten Ausführungsform einer erfindungsgemäßen Vorrichtung 1 zur Rekanalisierung eines zumindest teilweise verschlossenen Hohlraums bzw. Organwegs bzw. Gefäßes im menschlichen oder tierischen Körper. Die Vorrichtung umfasst einen Stent 10, der an seinem proximalen Ende 11 an einem Katheter 2 gehalten bzw. befestigt ist. Das proximale Ende des Stents 10 ist eingeschnürt ausgebildet bzw. verjüngt sich bis auf einen sehr geringen Durchmesser. In dieser Ausführungsform ist das proximale Ende 11 in eine Hülse 13 eingepresst. Hierdurch wird der Zusammenhalt des eingeschnürten proximalen Endes 11 des Stents sichergestellt. Außerdem kann die Hülse als eine Art Marker für das Verfolgen des Absetzvorgangs und der Position des Stents innerhalb des jeweiligen Hohlraums bzw. Organwegs bzw. Gefäßes innerhalb des menschlichen oder tierischen Körpers verwendet werden, wobei die Hülse unter dem Röntgenschirm sichtbar ist. Das Stentmaterial selbst kann röntgendicht sein. Der Katheter 2 ist mit seinem distalen Ende an dieser Hülse befestigt und weist vorzugsweise einen solchen Durchmesser auf, dass die Hülse darin gehalten werden kann, z.B. festgeklemmt ist. Die Hülse klemmt die proximalen Stentdrähte bzw. das proximale Ende des Stents auf das Katheterende.

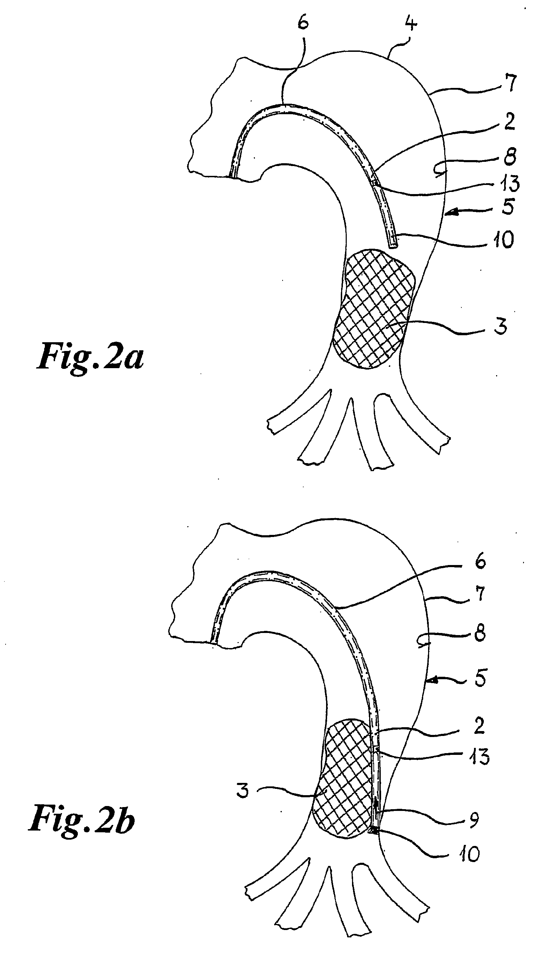

Der Stent selber weist eine Glockenform auf. Die Glockenform bedeutet, dass der Stent an seinem distalen Ende 12 auskragt, in Richtung zu seinem proximalen Ende im Bereich 15 im Durchmesser verringert ist, ausgehend von dem distalen Ende und nachfolgend einen aufgeweiteten Abschnitt 14 im proximalen Bereich aufweist, wobei sich der Stent ausgehend von dem aufgeweiteten Abschnitt 14 in Richtung zu dem proximalen Ende 11 wieder sehr stark verjüngt bzw. eingeschnürt ist. Der aufgeweitete Abschnitt 14 kann einen größeren oder etwa gleichen Durchmesser aufweisen wie das distale Ende 12 des Stents. Grundsätzlich ist es auch möglich, dass der aufgeweitete Abschnitt 14 einen etwas geringeren Durchmesser als das aufgeweitete distale Ende 12 aufweist. Der zwischen diesen beiden aufgeweiteten Abschnitten bzw. Enden angeordnete Bereich 15 ist gegenüber diesen verengt ausgebildet. Der Bereich mit dem geringsten Durchmesser ist vorzugsweise, wie in Figur 1 dargestellt, nahe dem distalen Ende 12 angeordnet. Hierdurch ist es besonders gut möglich, dass sich das distale Ende in einem Organweg bzw. Gefäß oder Hohlraum abstützen und festhalten kann. Zugleich kann ein zu komprimierendes Objekt dadurch gut von außen umfasst und an einem Herausgleiten gehindert werden. Dies ist besser den Figuren 2a bis 2f zu entnehmen. Eine weniger taillierte Ausführungsform eines solchen Stents ist in Figur 3 zu sehen, wobei dieser einen aufgeweiteten Bereich 14 mit im Vergleich zum Durchmesser des distalen Endes 12 etwa gleichem Durchmesser aufweist.

In den Figuren 2a bis 2f ist der Vorgang einer Rekanalisierung eines Organwegs am Beispiel einer Lungenembolie mit verschließendem Embolus dargestellt. Der Embolus 3 verschließt dabei einen Ast 5 einer Lungenarterie 4. Der Embolus sitz in einem Ast mit weiterem Durchmesser, so dass die Gefahr, die von einem solchen Embolus ausgeht, recht groß ist. Je größer der Embolus ist, desto größer ist auch das letale Risiko für den Patienten, da aufgrund der plötzlichen Erhöhung des Strömungswiderstandes durch den Embolus innerhalb der Lunge der Kreislauf zusammenbricht. Das rechte Herz muss das Blut, das sonst durch zwei Lungenarterien zur Lunge fließt, dann durch eine Lungenarterie pumpen. Hierdurch kann das rechte Herz sehr schnell überlastet werden und versagen. Sofern das rechte Herz der plötzlichen Mehrbelastung standhält, wird jedoch die Blutmenge vermindert, die die Lunge passieren und zum linken Herzen gelangen kann. Da das linke Herz nur so viel Blut weiterpumpen kann, wie bei ihm ankommt, führt eine herabgesetzte Fördermenge zu einer verminderten Sauerstoffversorgung der Organe, wohingegen sich das Blut vor dem rechten Herzen in den Venen anstaut und es gegebenenfalls zu einem Schock kommen kann. Daher ist es sehr wichtig, den Embolus so schnell wie möglich aus dem Ast der Lungenarterie zu entfernen bzw. diesen Ast wieder zu rekanalisieren.

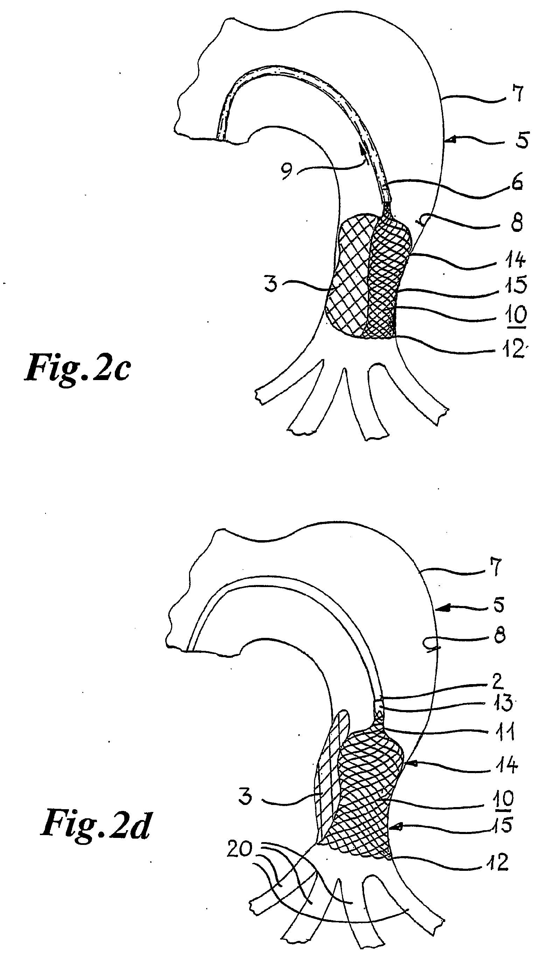

In Figur 2a ist daher ein Schleusenelement 6, z. B. ein Katheter, in die Lungenarterie 4 eingeführt. Innerhalb des Schleusenelements 6 wird der Katheter 2 zu dem Embolus 3 vorgeschoben. Der Stent 10 befindet sich zusammen mit dem Katheter 2 im komprimierten Zustand innerhalb des Schleusenelements 6 (gestrichelt in Figur 2a bis 2f dargestellt). Der Embolus 3 liegt so in der Lungenarterie, dass er allseitig an der Gefäßwandung 7 anliegt. Das Schleusenelement 6 wird zwischen Gefäßwand und Embolus vorbeigeschoben, wie dies insbesondere Figur 2b entnommen werden kann. Es kann über einen Führungsdraht dort eingeführt werden. Das Schleusenelement 6 wird dabei möglichst nicht durch den Embolus hindurch-, sondern an diesem vorbeigeschoben, um diesen besser komprimieren zu können. Als vorteilhaft erweist es sich, das Schleusenelement 6 bis an das distale Ende des Embolus zu schieben und erst dort mit dem Absetzvorgang für den Stent 10 zu beginnen. Hierdurch ist es möglich, dass das distale Ende 12 des Stents 10 sich an dem distalen Ende des Embolus vorbeischiebt und dadurch den Embolus von distal umgreift und gegen die Innenseite 8 der Gefäßwandung 7 drückt. Der Katheter 2 mit dem komprimierten Stent 10 wird durch das parallel zum Embolus liegende Schleusenelement vorgeschoben. Der Stent 10 wird mit kontrolliertem Zurückziehen des Schleusenelements, was durch den Pfeil 9 in Fig. 2b und 2c angedeutet ist, entfaltet. Da der Stent so ausgebildet ist, dass er selbst expandiert, drückt er nach dem Herausschieben aus dem Schleusenelement 6 den Embolus immer weiter gegen die Gefäßwand 7. Der vollständig entfaltete Zustand des Stents ist in Figur 2d gezeigt. Üblicherweise wird während des Rekanalisierungsvorgangs, der wenige Stunden bis Tage andauern kann, der Katheter mit dem Stent verbunden bleiben. Alternativ kann der Katheter aus der Lunge zurückgezogen und erst zum Entfernen des Stents wieder über das Schleusenelement dort eingeführt werden. In diesem Falle kann der Stent mit einer Verbindungseinrichtung zum Erleichtern des Wiederverbindens von Stent und Katheter 2 vorgesehen werden, beispielsweise einem Hakenelement an der Hülse 13 und einer Fangschlinge am distalen Ende des Katheters.

Wie Figur 2d zu entnehmen ist, führt die Glockenform des Stents dazu, dass der Embolus ohne die Möglichkeit eines Weiterrutschens in die nachfolgenden kleineren Arterienäste gegen die Gefäßwand gedrückt wird. Auch am proximalen Ende des Stents hat der Embolus keine Möglichkeit, wieder in den Durchflussweg der Arterie zu gelangen. Dies verhindert wirksam das eingeschnürte proximale Ende des Stents 10. Sogar, wenn beim Zusammenpressen des Embolus im Bereich des aufgeweiteten Abschnitts 14 nach proximal ein Abschnitt des Embolus abgetrennt würde, gelangte dieser nicht in die kleineren Lungenarterienäste 20, die in Figur 2d ebenfalls angedeutet sind. Würde ein solches abgetrenntes Teilstück des Embolus in diese Lungenäste gelangen, könnte es auch hier zu einer Embolie kommen, was insbesondere mit dem Stent des Standes der Technik gemäß WO02/064065 nicht vermieden werden kann.

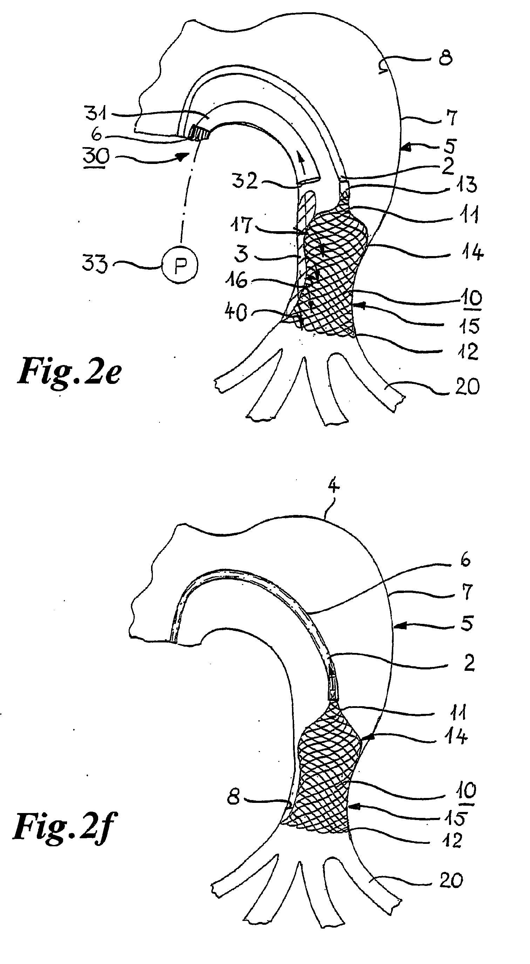

In Figur 2e wird die Wirkung des Stents 10 veranschaulicht. Dadurch, dass der Stent selbstexpandierend ist und einen im Wesentlichen gleichen oder etwas größeren Durchmesser als das zu rekanalisierende Gefäß aufweist, kann der Embolus durch das Pressen an die Gefäßwand ausgepresst werden. Dies bedeutet, dass flüssige Bestandteile durch die Maschen des Stents hindurch auf dessen Innenseite 16 gelangen und die festen Bestandteile auf der Außenseite 17 des Stents verbleiben. Die flüssigen Bestandteile werden mit dem durch den Stent hindurchfließenden Blut abtransportiert (Pfeile 40), wohingegen die festen Bestandteile zwischen der Gefäßwand 7 und der Außenseite 17 des Stents festgehalten werden. Ein Abtransport der festen Bestandteile über das Blut ist nach Entfernen des Stents einigermaßen problemlos möglich. Embolusreste an der Gefäßwand werden durch Eigenfibrinolyse (körpereigene Lyseagentien) der Lunge abgebaut. Zusätzlich kann zum Abbau der festen Bestandteile eine medikamentöse Lyse vorgesehen werden. Alternativ kann eine Absaugeinrichtung 30 zum Absaugen der festen Bestandteile in den Bereich des Embolus 3 durch ein Schleusenelement 6 vorgeschoben werden. Die Absaugeinrichtung weist in Figur 2e eine Kanüle 31 auf, die proximal von dem Embolus mit ihrer distalen Öffnung 32 positioniert wird. Proximal wird an der Absaugeinrichtung eine Pumpe 33 angeordnet, die einen Unterdruck erzeugt, wodurch die festen Bestandteile des Embolus über die distale Öffnung 32 in die Absaugeinrichtung bzw. die Kanüle 31 abgesogen werden können.

Nach dem Entfernen des Embolus bzw. dessen Ausdrücken wird das Schleusenelement 6 gegenüber dem gehaltenen Katheter 2 vorgeschoben, was zur Kompression des Stents führt, wonach dieser wieder aus der Lunge des Patienten entfernt werden kann. Dieser Vorgang ist in Figur 2f dargestellt. Beim Zurückziehen wird als letztes das auskragende distale Ende 12 des Stents komprimiert.

Um eine besonders gute Trennung der flüssigen und festen Bestandteile des Embolus durch den Stent vornehmen zu können, weist letzterer eine Netzstruktur auf. Diese kann z. B. durch ein Drahtgewebe bzw. Drahtgelege bzw. Drahtgeflecht gebildet werden. Alternativ kann auch ein geschnittenes Röhrchen mit einer solchen Struktur versehen werden. Wichtig sind dabei eine Anzahl von Öffnungen, um flüssige Bestandteile des Embolus oder allgemein des zu komprimierenden Objekts auf die Innenseite des Stents gelangen lassen zu können. Die Öffnungen sollten nicht zu groß sein, damit feste Bestandteile auf der Außenseite des Stents zwischen diesem und der Gefäßwand festgehalten werden können. Der Stent kann somit ein sehr feines Netzwerk aufweisen oder ein gröberes, wobei die Öffnungsweite der einzelnen Maschen kleiner sein sollte als der kleinste Durchmesser der Lungenäste. Hierdurch kann vermieden werden, dass feste Bestandteile sich in den nachfolgenden Lungenästen wieder festsetzen.

Durch das Aussetzen des Stents parallel zum Embolus kann dieser gegen die Gefäßwand gedrückt werden. Durch den Stent hindurch kann die Peripherie der nachfolgenden Lungenverästelungen wieder versorgt werden, womit die nachteiligen Folgen der Unterversorgung dieser Verästelungen und des Aufbaus eines hohen Drucks durch den Embolus aufgehoben werden können. Je nach Form des zu komprimierenden Objekts, insbesondere Embolus oder Thrombus, kann der Stent Stunden bis Tage in dem jeweils zu rekanalisierenden Hohlraum bzw. Gefäß bzw. Organweg verbleiben. Gerade bei kleineren, stark wasserhaltigen Verschlussobjekten wird die Verweilzeit des Stents in dem Hohlraum/Gefäß/Organweg geringer sein als bei einem großen, relativ festen Verschlussobjekt. Die erfindungsgemäße Vorrichtung zur Rekanalisierung eignet sich besonders für die Verwendung bei Lungenembolien mit verschließendem Embolus, da hier sehr schnell ein sehr großer Durchflussweg freigelegt werden kann. Da sich ein solcher Embolus relativ leicht ausquetschen lässt, sind die Verweilzeiten des Stents in der Lunge außerdem verhältnismäßig kurz, wobei nachfolgend kein operativer Eingriff zum Entfernen von Embolusmaterial mehr erforderlich ist, sondern nur minimal invasiv der Stent über ein Schleusenelement wieder entfernt wird.

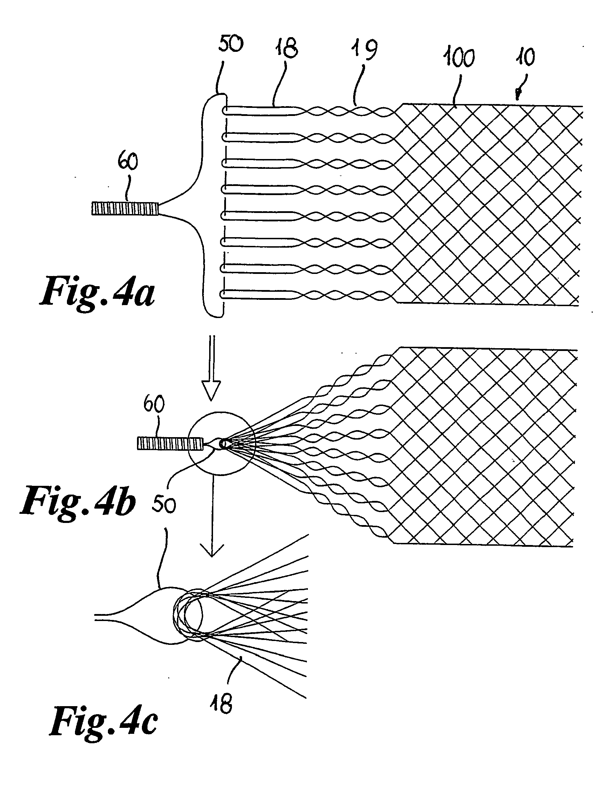

In den Figuren 4a-7c sind verschiedene Ausführungsformen einer alternativen Befestigungsart zum Befestigen des Stents proximal, also im Bereich des eingeschnürten Ende des Stents gezeigt. Anstelle des Auffügens eines Hülsenelementes bzw. einer Hülse ist dort nun eine Besfestigung über ein oder mehrere drahtartige Befestigungselemente vorgesehen. Gemäß Figur 4a sind proximal endseitig an der Netzstruktur Schlaufen 18 gebildet, die mit der Netzstruktur 100 des Stents 10 direkt verbunden bzw. aus dieser heraus gebildet sind. Hierbei werden jeweils am proximalen Ende der Netzstruktur zwei Fadenendabschnitte als Schlaufen herausgezogen und in einem Abschnitt 19 verdrillt.

Durch die Schlaufen 18 ist ein drahtartiges Befestigungselement 50 hindurchgefädelt, das proximal in einen Katheter 60 hineingeführt ist. Durch den Katheter hindurch kann es nach excorporal geführt und dort zum Halten des Stents festgelegt werden. Die Darstellung in Figur 4a zeigt die noch lockere Verbindung zwischen drahtartigem Befestigungselement 50 und Schlaufen 18, wohingegen die Detailansicht in Figur 4b den zusammengezogenen Zustand der Schlaufe des drahtartigen Befestigungselementes 50 und der diversen Schlaufen 18 des Stents zeigt. Die durch das drahtartige Befestigungselement 50 gebildete Schlaufe 51 ist besonders gut der vergrößerten Ansicht in Figur 4c zu sehen. Hierbei ist ebenfalls deutlich, dass die einzelnen Schlaufen 18 zusammengelegt ebenfalls eine Schlaufe bilden, durch die die Schlaufe 51 hindurchgreift. Die Einschnürung des proximalen Endes des Stents wird hierdurch erzeugt.

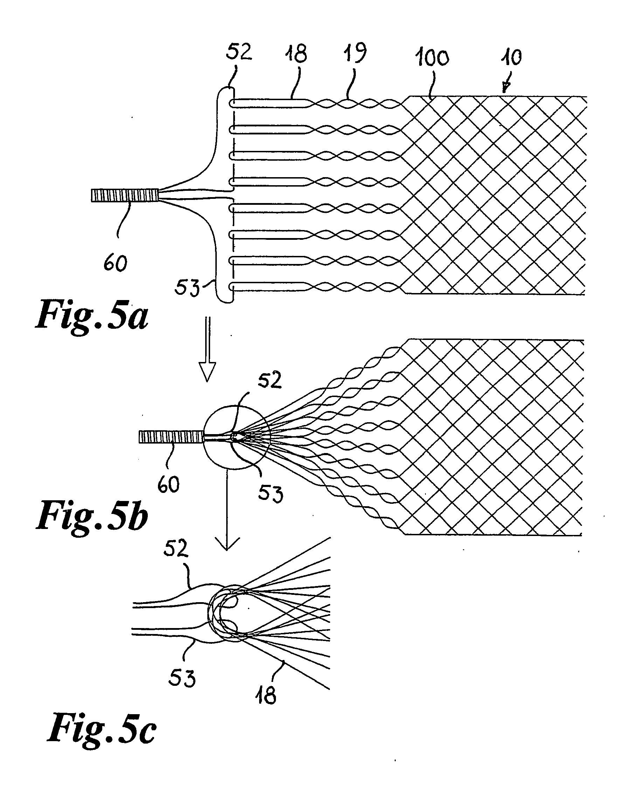

Bei der Ausführungsform der proximalen Befestigung des Stents in den Figuren 5a-5c sind anstelle des einen drahtartigen Befestigungselementes zwei drahtartige Befestigungselemente 52, 53 vorgesehen. Es sind an dem proximalen Ende des Stents ebenfalls wiederum Schlaufen 18 und verdrillte Abschnitte 19 aus der Netzstruktur 100 heraus gebildet. In dieser Ausführungsform greift das drahtartige Befestigungselement 52 lediglich durch die Hälfte der Schlaufen hindurch und fädelt diese auf, wohingegen das andere drahtartige Befestigungselement 53 durch die andere Hälfte der Schlaufen hindurchgreift und diese auffädelt. Der zusammengeschnürte bzw. zusammengeraffte Zustand der Schlaufen ist in Figur 5b gezeigt bzw. in der Detailansicht hierzu in Figur 5c. Hierbei ist deutlich, dass die Schlaufen 18 zwar wiederum eine große Schlaufe bilden, in diese jedoch die beiden kleineren Schlaufen der drahtartigen Befestigungselemente 52 und 53 hineingreifen. Anstelle des Vorsehens zweier solcher drahtartiger Befestigungselemente können grundsätzlich mehr als zwei vorgesehen werden, die jeweils in einen Teil der Schlaufen ein- und durch diese hindurchgreifen, um ein Einschnüren des proximalen Endes des Stents zu ermöglichen.

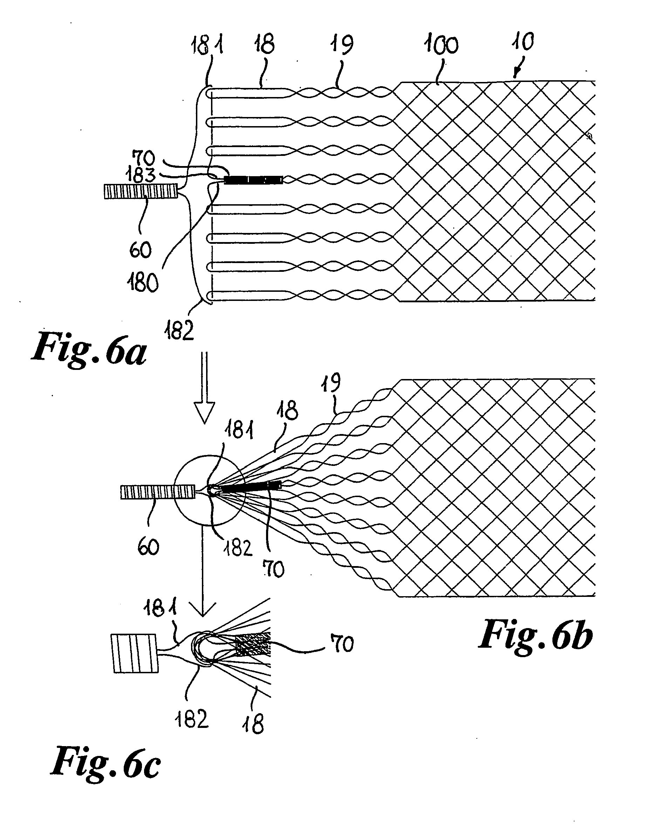

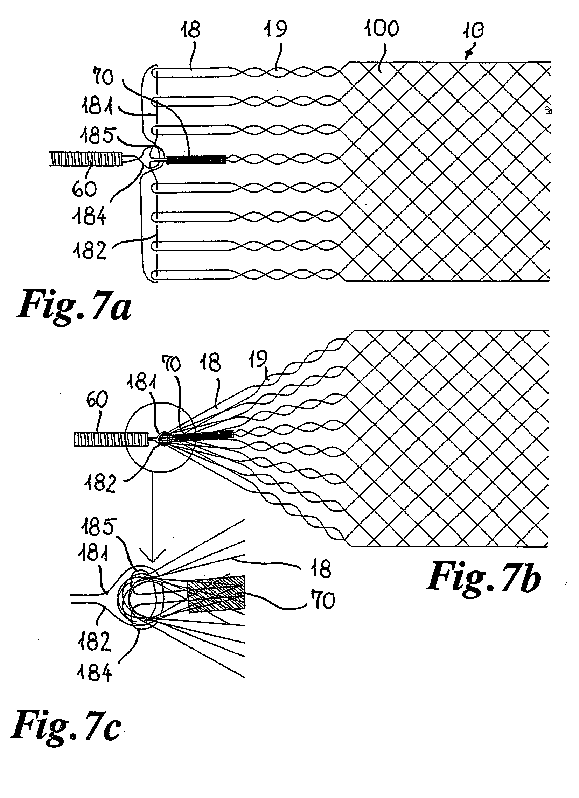

Eine weitere Alternative zu den Figuren 4 und 5 ist in den Figuren 6a - 7c gezeigt. Hier ist kein separates drahtartiges Befestigungselement vorgesehen, sondern eine der Schlaufen, nämlich die Schlaufe 180 länger als die anderen Schlaufen 18 ausgebildet, wobei wiederum die Schlaufe aus der Netzstruktur 100 des Stents herausgezogen und über einen verdrillten Abschnitt 19 zum proximalen Ende des Stents geführt ist. Die Schlaufe 180 ragt jedoch über das eigentliche proximale Ende des Stents hinaus und ist durch die übrigen Schlaufen des Stents mit ihren beiden Schlaufenteilen 181, 182 hindurchgefädelt. Die beiden Schlaufenteile 181, 182 sind nach dem Durchfädeln der übrigen Schlaufen 18 in den Katheter 60 hineingeführt und dienen genauso wie die drahtartigen Befestigungselemente zum proximalen Befestigen des Stents. Um eine Stabilisierung der Befestigung zu ermöglichen, gerade da die eine Schlaufe durch die übrigen hindurchgefädelt ist und dies ansonsten gegebenenfalls zu einer Verschiebung auch der Netzstruktur führen könnte, ist auf den Schlaufenabschnitt 183, der sich zwischen dem verdrillten Abschnitt 19 und den beiden Schlaufenteilen 181, 182 befindet, ein Hülsenelement 70 aufgefügt. Beim Zusammenziehen der Schlaufen durch Zurückziehen der Schlaufenteile 181, 182 in den Katheter 60 hinein kann das geordnete Zusammenziehen die Stabilisierung durch das Hülsenelement 70 besonders gut auch den Figuren 6b und der Detailansicht in Figur 6c entnommen werden. In ähnlicher Weise erfolgt auch in der Ausführungsform gemäß Figur 7a-c eine Stabilisierung über das Hülsenelement 70, wobei bei dieser Ausführungsform die beiden Schlaufenteile 181, 182 vor dem Einfügen in den Katheter 60 zunächst einmal noch in weitere Schlaufen 184, 185 gelegt sind, was beim Zusammenziehen der Schlaufen 18 zu der in den Figuren 7b und 7c gezeigten doppelten Umschlingung und damit besseren Befestigung der Schlaufen 18 im proximalen Bereich führt.

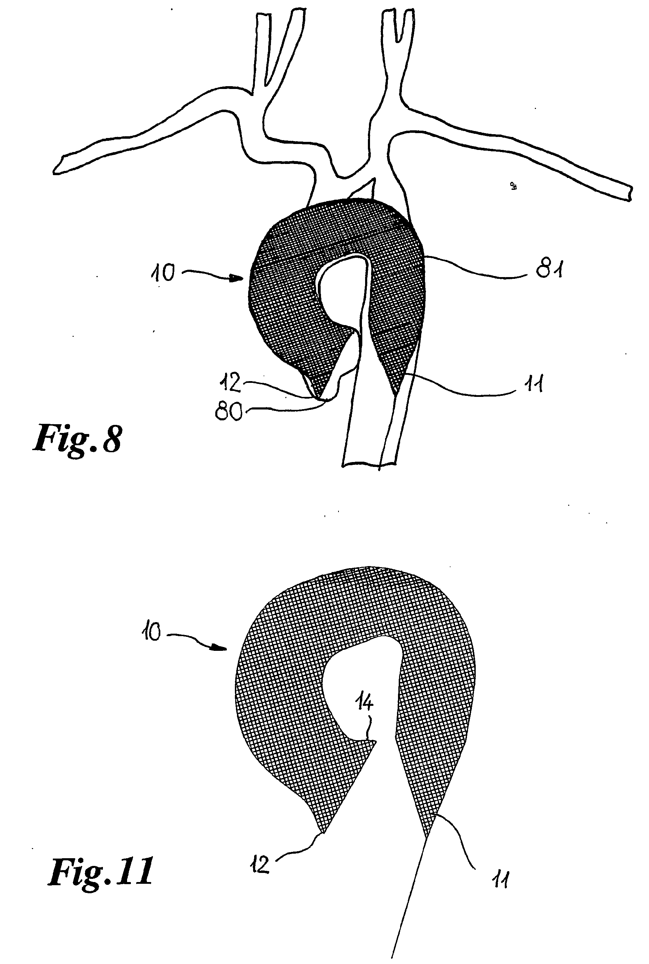

In den Figuren 8 und 11 ist jeweils ein langer erfindungsgemäßer Stent in der Anwendung als Aortenfilter gezeigt. In Figur 8 ist dabei der Aortenbogen im Hintergrund angedeutet, in Figur 11 der erfindungsgemäße Stent ohne den Aortenbogen dargestellt. Das distale Ende 12 des Stents 10 kann sich dabei in dem ausgestülpten Ende 80 des Aortenbogens 81 festhalten. Wie besonders gut in Figur 8 zu sehen, füllt der Stent nach der Implantation im Aortenbogen diesen nahezu vollständig aus, so dass eine gute Filterwirkung durch Drücken von unerwünschten Partikeln gegen die Wandungen des Aortenbogens erzielt werden kann. Ein solcher Aortenfilter kann besonders vorteilhaft für Patienten verwendet werden, die an einem hohen akuten Risiko eines Infarktes leiden, insbesondere bei Patienten mit diagnostiziertem Thrombus. Bei den meisten Patienten wird ein akutes hohes Risiko eines Thrombus im linken Herzohr diagnostiziert. Bei diesen kann ein erfindungsgemäßer Stent besonders gut und erfolgreich zur Filterung eingesetzt werden. Ein solcher Aortenfilter kann nicht nur frisch operierten Patienten helfen, sondern auch PFO-Patienten, die an einem massiven venösen Thrombus leiden. Auch Patienten mit angeborenen oder dilatierten Herzfehlern können besonders gut mit einer solchen Art von Aortenfilter behandelt werden. Besonders bevorzugt werden derartige nur temporär implantierte Filter, also Filter, die nach einer bestimmten Zeit wieder extrahiert werden, mit gerinnungshemmenden Medikamenten zusammen implantiert, die, wie bereits vorstehend ausgeführt, auch auf dem Stent selbst aufgebracht werden können.

Ein solcher Aortenfilter wird vor allem in der Embolusvorsorge zum Schutz der Koronarien (Herz) und der Karotis (Gehirn) verwendet. Zugleich wird er auch als Thrombektomiesystem für den Schutz vor Thromben in der aorta discendenta verwendet.

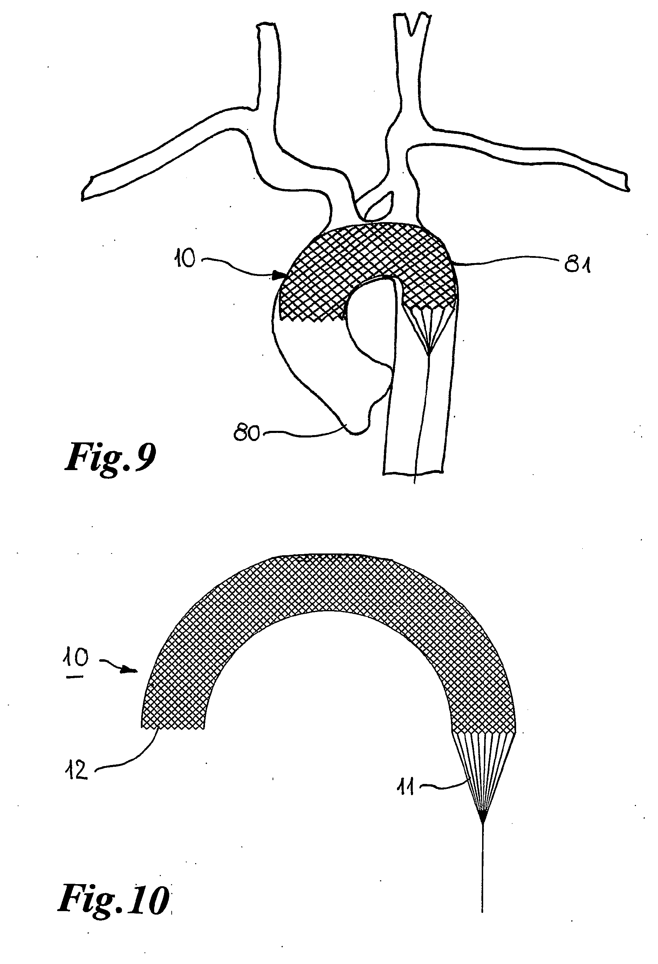

Anstelle des Vorsehens eines langen, sich bis zu dem ausgestülpten Ende des Aortenbogens erstreckenden Stents kann auch ein kürzerer Stent vorgesehen werden, wie beispielsweise in den Figuren 9 und 10 gezeigt. Die jeweiligen Stents ragen lediglich in einem 180°-Bogen um den am stärksten gekrümmten Teil des Aortenbogens herum. Das jeweilige distale Ende des kurzen Aortenfilterstents ist dabei im Wesentlichen nicht auskragend ausgebildet. Gleichwohl kann auch hier ein auskragender bzw. aufgeweiteter Abschnitt 14 vorgesehen werden. Es reicht jedoch bereits auch aus, lediglich ein gleichmäßig gerades Ende dort vorzusehen, sofern die Länge und Formgebung des Stents ein ungewolltes Bewegen desselben am Implantationsort verhindert.

Wie den Figuren 8 - 11 außerdem zu entnehmen ist, sind die proximalen Enden 11 jeweils durch ein drahtartiges Befestigungselement bzw. Schlaufenteile 181, 182 gehalten. Bei der Ausführungsform gemäß Figur 11 erstreckt sich die Netzstruktur bis zur proximalen Spitze, wohingegen bei der Ausführungsform gemäß Figur 10 lange Schlaufen gebildet und proximal zusammengefasst sind. Hierbei können auch beliebige Zwischenstufen ausgebildet werden, wobei selbstverständlich auch wiederum eine Befestigung über eine Hülse 13 und einen entsprechenden Katheter 2 möglich ist.

In dem im Vorstehenden beschriebenen und in den Figuren dargestellten Ausführungsformen einer Vorrichtung zur Rekanalisierung eines Hohlraums, Organwegs oder Gefäßes im menschlichen oder tierischen Körper können noch zahlreiche weitere Ausführungsformen gebildet werden, bei denen jeweils ein glockenförmiger Stent vorgesehen ist. Insbesondere kann die Form des Stents auf die Form des Hohlraums bzw. Organwegs bzw. Gefäßes angepasst werden. Teilweise eignen sich eher kürzere Stents mit einem größeren Öffnungsdurchmesser, bei anderen Anwendungsstellen können eher schmalere, längere Stents vorteilhaft sein. Bei jeder dieser Ausführungsformen ist ein Ende des Stents weit auskragend und geöffnet ausgebildet, wohingegen das gegenüberliegende Ende im Wesentlichen geschlossen und eingeschnürt ausgebildet ist, wobei benachbart zu diesem eingeschnürten Ende ein weiterer aufgeweiteter Bereich vorgesehen ist, um einen besonders guten Halt innerhalb des Hohlraums bzw. Organwegs bzw. Gefäßes im menschlichen oder tierischen Körper sicherzustellen.

### Bezugszeichenliste

- 1: Vorrichtung
- 2: Katheter
- 3: Embolus (Blutpfropf)
- 4: Lungenarterie
- 5: Ast der Lungenarterie
- 6: Schleusenelement
- 7: Gefäßwand
- 8: Innenseite
- 9: Pfeil
- 10: Stent
- 11: Proximales Ende
- 12: Distales Ende
- 13: Hülse
- 14: Aufgeweiteter Abschnitt
- 15: Verengter Bereich
- 16: Innenseite
- 17: Außenseite
- 18: Schlaufe, proximal
- 19: verdrillter Abschnitt
- 20: Lungenäste
- 30: Absaugeinrichtung
- 31: Kanüle
- 32: Distale Öffnung
- 33: Pumpe
- 40: Pfeil
- 50: drahtartiges Befestigungselement
- 51: Schlaufe
- 52: drahtartiges Befestigungselement
- 53: drahtartiges Befestigungselement
- 60: Katheter
- 70: Hülsenelement
- 80: ausgestülptes Ende des Aortenbogens
- 81: Aortenbogen
- 100: Netzstruktur
- 180: Schlaufe
- 181: Schlaufenteil
- 182: Schlaufenteil
- 183: Schlaufenabschnitt
- 184: Schlaufe
- 185: Schlaufe

## Patentansprüche

1. Vorrichtung (1) zur Rekanalisierung eines von zumindest einem komprimierbaren Verschlussobjekt, insbesondere einem Thrombus oder Embolus (3), zumindest teilweise verschlossenen Hohlraums, Organwegs oder Gefäßes (4), im menschlichen oder tierischen Körper mit zumindest einem komprimierbaren und selbstexpandierenden Stent (10) mit einem distalen (12) und einem proximalen Ende (11),
**dadurch gekennzeichnet, dass**
der zumindest eine Stent (10) im Wesentlichen glockenförmig mit einem aufgeweiteten distalen Ende (12) und einem eingeschnürten proximalen Ende (11) ausgebildet ist, wobei ein zwischen dem distalen (12) und dem proximalen Ende (11) des zumindest einen Stents (10) angeordneter Bereich (15) gegenüber der Öffnungsweite des distalen Endes (12) verengt und ein zwischen dem verengten Bereich (15) und dem eingeschnürten proximalen Ende (11) angeordneter Abschnitt (14) gegenüber dem verengten Bereich (15) aufgeweitet ist.

2. Vorrichtung (1) nach Anspruch 1,
**dadurch gekennzeichnet, dass**
das eingeschnürte proximale Ende (11) des Stents (10) mit einem Hülsenelement (13) versehen ist.

3. Vorrichtung (1) nach einem der vorstehenden Ansprüche,
**dadurch gekennzeichnet, dass**
der Stent (10) eine Netzstruktur und/oder eine Vielzahl von Öffnungen mit einer Öffnungsweite, die geringer als die Abmessungen des zu komprimierenden Verschlussobjekts (3) ist, aufweist.

4. Vorrichtung nach Anspruch 3,
**dadurch gekennzeichnet, dass**
der Stent (10) aus einem Geflecht und/oder Gewebe und/oder Gelege besteht, insbesondere einem Drahtgeflecht und/oder Drahtgewebe und/oder Drahtgelege.

5. Vorrichtung (1) nach Anspruch 3,
**dadurch gekennzeichnet, dass**
der zumindest eine Stent aus einem zumindest über einen Teil seiner Länge geschlitzten Rohr besteht.

6. Vorrichtung (1) nach einem der vorstehenden Ansprüche,
**dadurch gekennzeichnet, dass**
das Material des zumindest einen Stents (10) mit einer Beschichtung versehen ist, insbesondere einer biokompatiblen Oberflächenbeschichtung, Heparin, einer Karbonisierung von Nitinol, einer nanotechnologischen Beschichtung, röntgendichten Partikeln, einer einen Wirkstoff freisetzenden Beschichtung, einer insbesondere mikroporösen biotechnologischen oder einer anderen Beschichtung.

7. Vorrichtung (1) nach einem der vorstehenden Ansprüche,
**dadurch gekennzeichnet, dass**
der zumindest eine Stent (10) aus einem biokompatiblen Formgedächtnismaterial, insbesondere einem Metall oder einer Metalllegierung, insbesondere einem Edelstahl oder Nitinol oder einem anderen biokompatiblen Material, insbesondere einem Kunststoff besteht.

8. Vorrichtung (1) nach einem der vorstehenden Ansprüche,
**dadurch gekennzeichnet, dass**
der Durchmesser des expandierten Stents (10) im Wesentlichen gleich dem oder etwas größer als der Durchmesser des zu rekanalisierenden Hohlraums, Organswegs oder Gefäßes (4) dimensioniert ist.

9. Vorrichtung (1) nach einem der vorstehenden Ansprüche,
**dadurch gekennzeichnet, dass**
eine Absaugeinrichtung (30) zum Absaugen von festen Bestandteilen des zu komprimierenden Verschlussobjekts (3) oder Objektteilen vorgesehen ist, insbesondere eine Kanüle (31) oder dergleichen rohrförmige Einrichtung, die in die Nähe des zumindest einen in dem Hohlraum, Organweg oder Gefäß (4) abgesetzten Stents (10) führbar und mit einem Unterdruck beaufschlagbar ist.

10. Komprimierbarer und selbstexpandierender Stent mit einem distalen (12) und einem proximalen Ende (11), nach einem der Ansprüche 1 bis 9,
**dadurch gekennzeichnet, dass**
zum Befestigen des Stents (10) ein oder mehrere drahtartige Befestigungselemente (50,52,53) vorgesehen sind, wobei das proximale Ende (11) des Stents (10) mit zumindest teilweise verdrillten Schlaufen (18, 180) ausgebildet ist.

11. Stent nach Anspruch 10,
**dadurch gekennzeichnet, dass**
die drahtartigen Befestigungselemente (50,52,53) Einzeldrähte oder eine Drahtlitze sind.

12. Stent nach Anspruch 11,
**dadurch gekennzeichnet, dass**
die zumindest teilweise verdrillten Schlaufen (18, 180) aus einer Netzstruktur (100) des Stents (10) herausgeführt sind.

13. Stent nach Anspruch 12,
**dadurch gekennzeichnet, dass**
alle oder ein Teil der Schlaufen (18) von dem oder den drahtartigen Befestigungselementen (50, 52, 53) zusammengefasst oder zusammenfassbar ist.

14. Stent nach Anspruch 12 oder 13,
**dadurch gekennzeichnet, dass**
zumindest eine zumindest teilweise verdrillte Schlaufe (180) länger als die übrigen Schlaufen (18) ausgebildet und durch diese hindurchgefädelt ist zum Ausbilden eines drahtartigen Befestigungselements für den Stent (10).

15. Stent nach Anspruch 14,
**dadurch gekennzeichnet, dass**
die als drahtartiges Befestigungselement ausgebildete Schlaufe (180) endseitig geschlossen oder offen ausgebildet ist.

16. Stent nach Anspruch 14 oder 15,
**dadurch gekennzeichnet, dass**
die als drahtartiges Befestigungselement ausgebildete Schlaufe (180) im Bereich zwischen ihrem verdrillten Abschnitt (19) und dem durch die benachbarten Schlaufen (18) gefädelten Abschnitt (181, 182) mit einem Hülsenelement (70) zum Stabilisieren versehen ist.

## Claims

1. A device (1) for rechanneling a cavity, an organ path or a vessel which is at least partially closed by at least one compressible occlusive object, in particular a thrombus or embolus (3) in the human or animal body, comprising at least one compressible and self-expanding stent (10) having a distal (12) and a proximal end (11),
**characterized in that**
the at least one stent (10) is essentially bell-shaped with an expanded distal end (12) and a constricted proximal end (11), wherein a portion (15) which is placed between the distal (12) and the proximal end (11) of the at least one stent (10) is narrowed with respect to the opening width of the distal end (12) and a portion (14) which is placed between the narrowed portion (15) and the constricted proximal end (11) is expanded with respect to the narrowed portion (15).

2. A device (1) according to claim 1,
**characterized in that**
the constricted proximal end (11) of the stent is provided with a sleeve element (13).

3. A device (1) according to one of the preceding claims,
**characterized in that**
the stent comprises a reticular structure and/or a multiplicity of openings having an opening width which is smaller than the dimensions of the occlusive object (3) to be compressed.

4. A device according to claim 3,
**characterized in that**
the stent (10) is made of a braided material and/or woven material and/or scrim, in particular a wire braid and/or woven wire material and/or wire scrim.

5. A device (1) according to claim 3,
**characterized in that**
the at least one stent consists of a tube which is slotted at least along part of its length.

6. A device (1) according to one of the preceding claims,
**characterized in that**
the material of the at least one stent (10) is provided with a coating, in particular a biocompatible surface coating, heparin, a carbonization of nitinol, a nanotechnological coating, radiopaque particles, a coating releasing an active substance, an in particular microporous biotechnological or other coating.

7. A device (1) according to one of the preceding claims,
**characterized in that**
the at least one stent (10) consists of a biocompatible shape-memory-effect material, in particular a metal or a metal alloy, in particular a stainless steel or nitinol or another biocompatible material, such as a plastic in particular.

8. A device (1) according to one of the preceding claims,
**characterized in that**
the diameter of the expanded stent (10) is substantially equal to or slightly larger than the diameter of the cavity, organ path or vessel (4) to be rechaneled.

9. A device (1) according to one of the preceding claims,
**characterized in that**
a suction device (30) for suctioning off solid constituents of the occlusive object to be compressed or parts of the object is provided, in particular a cannula (31) or suchlike tubular device, which can be guided adjacent to the at least one stent (10) deployed in the cavity, organ path or vessel (4) and operated at subatmospheric pressure.

10. A compressible and self-expanding stent having a distal (12) and a proximal end (11) according to one of the claims 1 through 9,
**characterized in that**
one or more wire-like attachment elements (50, 52, 53) are provided for attachment of the stent, wherein the proximal end (11) of the stent (10) is designed with at least partly twisted loops (18, 180).

11. A stent according to claim 10,
**characterized in that**
the wire-like attachment elements (50, 52, 53) are single wires or a stranded wire.

12. A stent according to claim 11,
**characterized in that**
the at least partly twisted loops (18, 180) are passed out of a reticular structure (100) of the stent (10).

13. A stent according to claim 12,
**characterized in that**
all or only part of the loops (18) are or can be held together by the wire-like attachment element(s) (50, 52, 53).

14. A stent according to claim 12 or 13,
**characterized in that**
at least one at least partly twisted loop (180) is longer than the other loops (18) and threaded through these ones to form a wire-like attachment element for the stent (10).

15. A stent according to claim 14,
**characterized in that**
the loop (180) designed as wire-like attachment element is closed or open at its end.

16. A stent according to claim 14 or 15,
**characterized in that**
the loop (180) designed as wire-like attachment element is provided with a sleeve element (70) in the region between its twisted section (19) and the section (181, 182) threaded through the adjacent loops, for the purpose of stabilization.

## Revendications

1. Dispositif (1) de recanalisation d'une cavité, d'un canal organique ou d'un vaisseau qui est au moins partiellement clos par au moins un objet occlusif compressible, notamment un thrombus ou un embolus (3) dans le corps humain ou animal, comprenant au moins un stent (10) compressible et auto-expansif qui a une extrémité distale (12) et une extrémité proximale (11),
**caractérisé en ce que**
l'au moins un stent (10) est essentiellement configuré sous forme d'une cloche comprenant une extrémité distale élargie (12) et une extrémité proximale serrée (11), une partie (15) disposée entre l'extrémité distale (12) et l'extrémité proximale (11) de l'au moins un stent (10) étant rétrécie par rapport à la largeur d'ouverture de l'extrémité distale (12) et une partie (14) disposée entre la partie rétrécie (15) et l'extrémité proximale serrée (11) étant élargie par rapport à la partie rétrécie (15).

2. Dispositif (1) selon la revendication 1,
**caractérisé en ce que**
l'extrémité proximale serrée (11) du stent (10) est munie d'un élément de gaine (13).

3. Dispositif (1) selon l'une des revendications précédentes,
**caractérisé en ce que**
le stent (10) comprend une structure réticulaire et/ou une multiplicité d'ouvertures ayant une largeur d'ouverture qui est moins grande que les dimensions de l'objet occlusif (3) à comprimer.

4. Dispositif selon la revendication 3,
**caractérisé en ce que**
le stent (10) est composé d'une matière tressée et/ou d'une matière tissée et/ou d'un tissu non tissé à couches superposées biaises, notamment d'un treillis en fil métallique et/ou d'un tissu métallique et/ou d'un tissu non tissé à fils métalliques superposés.

5. Dispositif (1) selon la revendication 3,
**caractérisé en ce que**
l'au moins un stent consiste en un tuyau qui est fendu le long d'au moins une partie de sa longueur.

6. Dispositif (1) selon l'une des revendications précédentes,
**caractérisé en ce que**
la matière de l'au moins un stent (10) est munie d'un revêtement, notamment d'un revêtement de surface biocompatible, d'héparine, d'une carbonisation de nitinol, d'un revêtement nano-technologique, des particules radiopaques, d'un revêtement qui dégage une matière active, d'un revêtement notamment microporeux biotechnologique ou d'un autre revêtement.

7. Dispositif (1) selon l'une des revendications précédentes,
**caractérisé en ce que**
l'au moins un stent (10) consiste en un matériau à effet de mémoire de forme biocompatible, notamment en un métal ou en un alliage de métal, notamment en un acier inoxydable ou en nitinol ou en un autre matériau biocompatible, tel que notamment en une matière artificielle.

8. Dispositif (1) selon l'une des revendications précédentes,
**caractérisé en ce que**
le diamètre du stent (10) expansé est essentiellement égal au ou un peu plus grand que le diamètre de la cavité, du canal organique ou du vaisseau (4) à recanaliser.

9. Dispositif (1) selon l'une des revendications précédentes,
**caractérisé en ce qu'**
un dispositif d'aspiration (30) est prévu pour aspirer des constituants solides de l'objet occlusif (3) à comprimer ou des parts d'objet, notamment une canule (31) ou un dispositif tubulaire similaire qu'on peut guider à proximité de l'au moins un stent (10) déployé dans la cavité, le canal organique ou le vaisseau (4) et qu'on peut opérer à une pression subatmosphérique.

10. Stent compressible et auto-expansif comprenant une extrémité distale (12) et une extrémité proximale (11) selon l'une des revendications 1 à 9,
**caractérisé en ce qu'**
un ou plusieurs éléments de fixation (50, 52, 53) similaires à des fils métalliques sont prévus pour fixer le stent (10), l'extrémité proximale (11) du stent (10) étant configurée avec des boucles (18, 180) au moins partiellement torsadées.

11. Stent selon la revendication 10,
**caractérisé en ce que**
les éléments de fixation (50, 52, 53) similaires à des fils métalliques sont des fils métalliques individuels ou un toron métallique.

12. Stent selon la revendication 11,
**caractérisé en ce que**
les boucles (18, 180) au moins partiellement torsadées sont guidées vers l'extérieur d'une structure réticulaire (100) du stent (10).

13. Stent selon la revendication 12,
**caractérisé en ce que** l'ensemble ou une partie des boucles (18) est réuni ou peut être réuni par les éléments de fixation (50, 52, 53) similaires à des fils métalliques.

14. Stent selon la revendication 12 ou la revendication 13,
**caractérisé en ce qu'**
au moins une boucle (180) au moins partiellement torsadée est plus longue que les autres boucles (18) et est enfilée à travers de celles-ci pour former un élément de fixation similaire à un fil métallique du stent (10).

15. Stent selon la revendication 14,
**caractérisé en ce que**
la boucle (180) configurée comme un élément de fixation similaire à un fil métallique est fermée ou ouverte à son extrémité.

16. Stent selon la revendication 14 ou la revendication 15,
**caractérisé en ce que**
la boucle (180) configurée comme un élément de fixation similaire à un fil métallique est munie d'un élément de gaine (70) dans la région entre sa partie torsadée (19) et la partie (181, 182) enfilée à travers des boucles adjacentes, l'élément de gaine étant destiné à la stabilisation.
